# EUROPEAN PATENT SPECIFICATION

(11) **EP 2 958 615 B1**
(45) Date of publication and mention of the grant of the patent: **30.08.2017**
(21) Application number: 14714351.5
(22) Date of filing: 17.02.2014
(51) Int. Cl.: A61M 25/00, A61M 25/04, A61M 25/10

(54) **CATHETER FOR ADMINISTERING ACTIVE AGENTS**
KATHETER ZUR VERABREICHUNG VON WIRKSTOFFEN
CATHÉTER POUR ADMINISTRER DES AGENTS ACTIFS

(30) Priority: 19.02.2013 IT TO20130135
(43) Date of publication of application: 30.12.2015
(73) Proprietor: CID S.p.A., 13040 Saluggia (VC) (IT)
(72) Inventor: MINOLETTI, Francesco, I-10149 Torino (IT); SHIN, Dong Ik, Poway, California 92064 (US); VALLANA, Franco, I-10123 Torino (IT)
(74) Representative: Freyria Fava, Cristina
(86) International application number: PCT/IB2014/059050
(87) International publication number: WO 2014/128608

(56) References cited:
- WO-A1-2010/125166
- WO-A2-00/67832
- WO-A2-2004/045672
- WO-A2-2006/065949
- WO-A2-2012/151396
- US-A1- 2007 219 464
- US-A1- 2010 069 882
- US-A1- 2010 318 067
- US-A1- 2013 035 609
- US-B1- 6 692 466

## Description

### Technical field

The present description relates to catheters. Various embodiments can relate to catheters for administering active agent, for example, for administering medication to a tissue lesion.

### Technologycal background

The techniques of Percutaneous Transluminal Angioplasty (PTA) have been well established for years for treating stenotic vessels, such as the coronary arteries. In this context, the importance has been revealed of ensuring that the beneficial effects of the treatment are able to last over time.

For this reason, in addition to the plain and simple treatments of PTA, based on a sequence of dilatation actions of the stenotic vessel, carried out using a balloon catheter, there has been widespread use of techniques which involve an implant at the stenotic site, of a stent, which is an expandable tubular structure able to produce a support action of the vessel wall in order to permanently preserve the patency conditions achieved/restored with angioplasty.

The stent implantation does not, however, completely exclude the risk of restenosis phenomena. This has led to the development of a Drug Eluting Stent (DES), capable of releasing an active agent on the treated site, such as a restenosis-antagonist agent.

Balloon catheters capable of delivering an active agent, such as a drug, are known for other means, thanks to the fact that the wall of the balloon is provided with openings (or rather is "porous"). These openings allow a liquid placed inside the balloon itself to spread to the outside of the balloon, which serves as a liquid carrier for the active agent. In particular, the liquid in question may be the pressurized liquid used for dilating the balloon on the site subjected to treatment.

Moreover, catheters have been proposed for administering - by injection - a drug suitable for treating a tissue lesion, for example, a blood vessel or a tissue that is located in the vicinity of a blood vessel as demonstrated in the documents US-A-2007/0282257, WO-A-2012/029535, US-A-5 354 279, US-A-2011/0166516, US-A-5 693 029 and US-A-2003/0171714. Among other documents of interest one may cite US 2003/318067, US 2013/035609, WO 2010/125166, WO 00/67832, US 6 692 466, WO 2012/151396, WO 2004/045672, WO 2006/065949, US 200/069882, US 2007/219464.
In particular, the U.S. patent application US-A-2003/0171714, on which the preamble of claim 1 has been patterned, proposes a catheter equipped with a balloon and a needle for administering a drug to a cardiac lesion, where the balloon has the function of retaining the needle in the required position for administering the drug to the cardiac lesion.

The catheter described in US-A-2003/017174 presents, however, a relatively complex configuration for the expulsion of the needle from the catheter body, where it is the structure of the catheter body that determines the acquisition of the curvature required by the needle for the injection of the drug into the lesion.

### Object and summary

Various embodiments aim to provide a catheter suitable for administering an active agent to a body site, for example, for administering a drug to a deep lesion in a tissue, the catheter having a simple structure and being economically more advantageous to produce.

Various embodiments enable achievement of this objective thanks to a catheter having the characteristics referred to specifically in the claims that follow.

The claims form an integral part of the technical disclosure herein provided in relation to the invention.

Various embodiments exemplified here may refer to a percutaneously-insertable catheter for administering an active agent to a body site, the catheter comprising:
- a distal portion expandable from a contracted condition of introduction to a deployed administering condition, to stabilize the distal end of the catheter at said body site, and
- a flexible tubular element coupled to said distal end of the catheter to receive and administer the active agent to said body site.

In various embodiments, the flexible element may comprise an outer tubular element and an inner tubular element inserted into the outer tubular element, with one of said outer tubular element and said inner tubular element having notches, which allow the spinal column-like curvature towards said body site.

Various embodiments exemplified here may enable the administering of an active agent, such as a drug in either liquid form for a rapid uptake, or in gel-form to obtain a slower release profile, which is controlled by the lesion.

### Brief description of the figures

Embodiments will now be described, purely by way of non-limiting example, with reference to the attached figures, wherein:
- Figure 1 shows a catheter according to one embodiment;
- Figures 2 to 4 are sections along the lines II-II, III-III and IV-IV of Figure 1, respectively;
- Figure 5, comprising two parts indicated respectively by A and B, represents an embodiment of a flexible element shown in the retracted condition within the catheter (part A of Figure 5) and in the extracted condition from the catheter (part B of Figure 5) ;

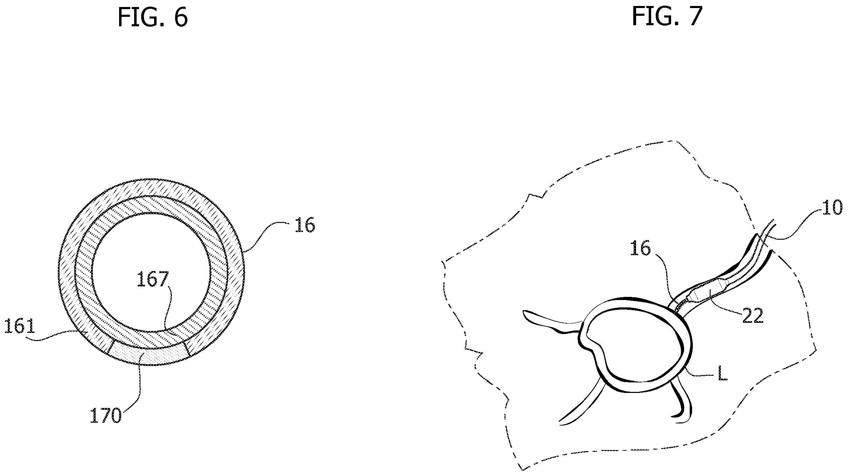

- Figure 6 is a section along the line VI-VI of part A of Figure 5; and
- Figure 7 shows the mode of use of embodiments.

### Detailed description

In the following description, various specific details are illustrated, aimed at a thorough understanding of various examples of embodiments. The embodiments can be implemented without one or more of the specific details, or with other methods, components, materials, etc. In other cases, known structures, materials, or operations are not shown or described in detail to avoid obscuring the various aspects of the embodiments.

The reference to "an embodiment" in the context of this description indicates that a particular configuration, structure or characteristic described in relation to the embodiment is included in at least one embodiment. Therefore, phrases such as "in an embodiment", possibly present in various points of this description do not necessarily refer to the same embodiment. Furthermore, particular conformations, structures, or characteristics may be combined in any suitable manner in one or more embodiments.

The references used herein are only for the convenience of the reader and therefore do not define the field of protection or the scope of the embodiments.

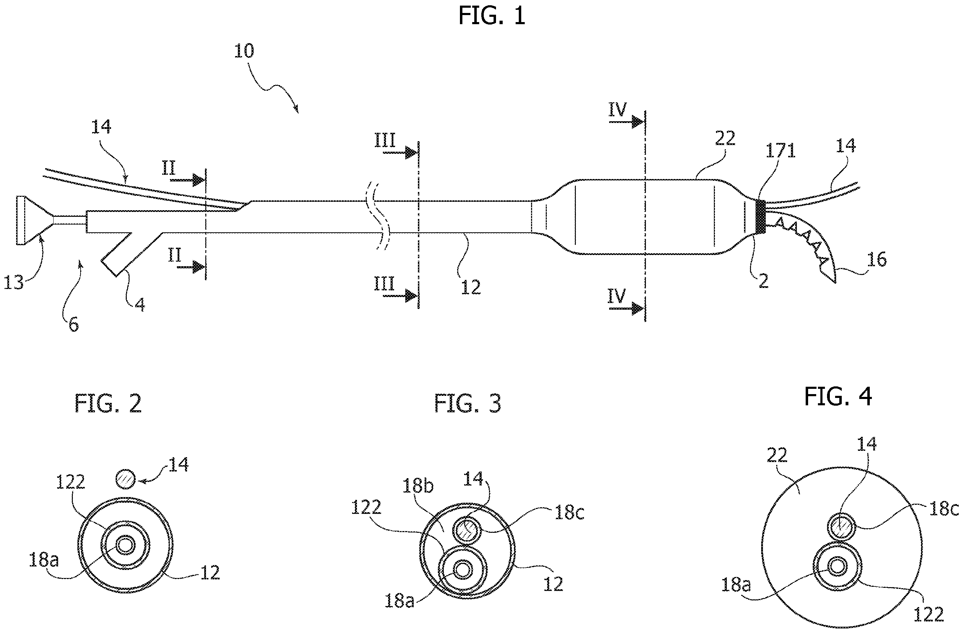

Figures 1 and 2 to 4 show, respectively, a side view and a series of cross-sectional views of a catheter 10, which can be used to administer an active agent to a body site, for example, to administer a drug to a tissue lesion.

In various embodiments, the catheter 10 can include a body 12 provided at its distal end 2 with a flexible (curvable) element 16 for administering an active agent (e.g. a drug, reagent, etc.) and an expandable portion 22, on a distal portion of the catheter 10, capable of being expanded between a contracted position and a deployed position.

In various embodiments, the expandable portion 22 can comprise an expandable balloon.

In various embodiments, a guide wire 14 can be associated with the catheter 10, along its entire length.

In various embodiments, the active agent (e.g. drug) can be supplied in liquid form, or as a gel, to the flexible element 16 through a supply lumen 18a, which extends within the body 12 of the catheter, and is connected - at the proximal end 6 of the catheter 10 - by means of a luer fitting 13 - to a system for supplying and pressurising the active agent to be administered (not visible in the figures, and of known type).

In various embodiments, the supply lumen 18a can be housed within an inner tubular sheath 122, which extends within the internal body 12 of the catheter 10.

The expandable portion (balloon) 22 is inflatable thanks to a fluid made to flow through a supply inlet 4 positioned at the proximal end 6 of the catheter itself, through an inflation lumen 18b, by a system for supplying and pressurising the inflation fluid (not visible in the figures, and of known type).

In various embodiments, the guide wire 14 and/or the flexible element 16 can be movable relative to the body 12 in the longitudinal direction.

In various embodiments, the flexible element 16 can be free to rotate around its own axis, thanks to a rotation system (not visible in the figures, and of known type) positioned at the proximal end of the catheter 10.

In various embodiments, the body 12 can have a third lumen 18c for sliding the guide wire 14.

In various embodiments, the three lumen 18a, 18b and 18c, and the inner tubular sheath 122 can extend longitudinally within the body 12 of the catheter and/or be independent of each other.

In various embodiments, the body 12 of the catheter 10 can have a diameter and a length so that they can be inserted into a blood vessel of the femoral region or of the brachial region of a human body, so as to carry the distal end of the catheter 10 to the area where the administering of an active agent is required, for example to the heart (percutaneous insertion).

In various embodiments, as exemplified here, the body 12 of the catheter can be created with a flexible material so that it can also be inserted into a tortuous blood vessel.

In various embodiments, a radiopaque element 171 (e.g. of platinum) can be associated with the distal end 2 of the catheter 10, having the function of terminal marker to allow the operator - by means of scopy - to appropriately orient the catheter 10 itself.

In various embodiments, the expandable portion (balloon) 22 can be used to stabilize the distal end 2 of the catheter in order to enable the flexible element 16 to penetrate into the vessel wall to inject the active agent supplied through the conduit 18a. In various embodiments, the element 16 is capable of acting by flexible needle (curvable) for administering, in practice by injecting it in the site to be treated (e.g. into a tissue lesion), the active agent (drug, reagent, etc.) supplied through conduit 18a.

In various embodiments, this stabilizing function of the flexible element (needle) 16 can be conducted in addition to, or alternatively to, using the balloon 22 for carrying out angioplasty.

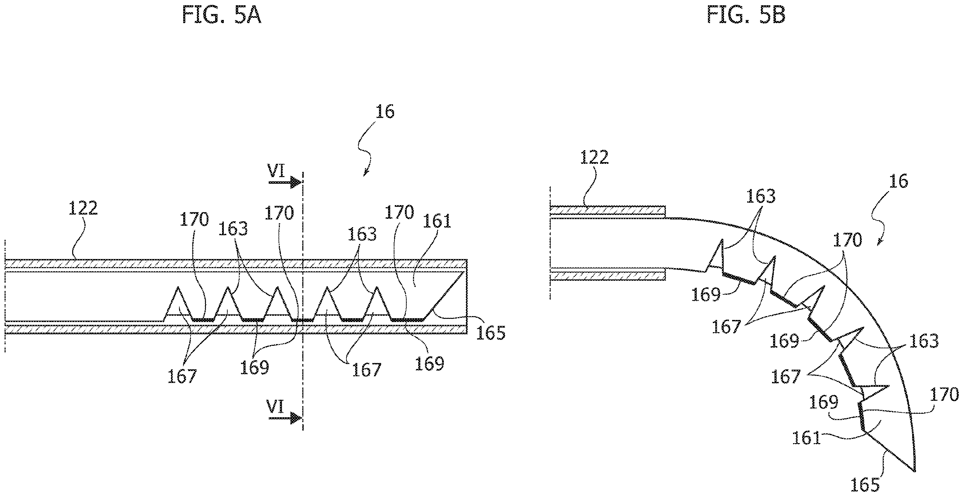

As exemplified in Figure 5, in various embodiments, the flexible element 16 can comprise a (first) outer tubular element 161 and a (second) inner or core tubular element 167 arranged within the first tubular element 161 and capable of receiving the active agent to be administered from the conduit 18a.

In various embodiments, the outer tubular element 161 can comprise a (super)elastic metallic material, such as Nitinol, while the inner or core tubular element 167 can comprise an flexible synthetic material (such as a polyamide resin or polyethylene).

In various embodiments, the first tubular element 161 can be provided with, on a portion of its lateral surface, a sequence of notches or slots 163, capable of allowing the flexible element 16 to become curved with a jointed configuration, roughly definable as "spinal column-like."

In other words, in various embodiments, the first tubular element 161 can comprise a plurality of open or closed sections or tubular segments ("vertebrae"), connected - at the bottom parts of the notches 163 - by flexible bridges 169 that enable bending of the element 161 itself.

Regarding this, it will be appreciated, that even when - according to various embodiments - the element 161 comprises a single piece of flexible material (e.g. an elastic or superelastic material such as nitinol), the tubular shape causes the aforementioned sections or segments to become rigid, or essentially rigid, with respect to bending in the longitudinal direction, and in any case, less flexible relative to the bridges 169 connecting them, these bridges 169 identifying the regions of longitudinal bending of the element 161.

In various embodiments, it is thus possible to ensure a general flexibility of the element 161 even if the individual portions of this element (i.e. the "vertebrae" that form the element) included between successive notches 163 are themselves quite stiff.

In various embodiments, the first tubular element 161 (and therefore the element 16 as a whole, thanks to the overall flexibility of the core element 167) can thus achieve, after extraction from the catheter 10, an overall curved configuration in order to allow its penetration at the site (e.g. injured tissue) to be treated.

In various embodiments, the flexible element 16 can present a sharp tip part (for example a chamfer cut 165 at the distal opening of the first tubular element 161), capable of facilitating the penetration of the flexible element 16 into the treated site.

In various embodiments, the notches 163 in the first tubular element 161 can be produced by means of laser technology and/or can generally present a V-profile.

In various embodiments, the notches 163 can be:
- the same or different from each other, and/or
- distributed uniformly or non-uniformly with the possibility to establish, according to the requirements of use, the arcuate shape that the tubular element 161 (and the element 16 as a whole) can assume after extraction from the body 12 of the catheter.

In various embodiments, the notches 163 can be, for example:
- aligned along a side (a generatrix) of the element 161 so as to be, when the element is curved, on the intrados of the arc of curvature or bending,
- aligned along two sides or opposite generatices of the element 161 so as to be, when the element is curved, in part on the intrados and in part on the extrados of the arc of curvature, and/or
- variously distributed on the element 161.

In various embodiments, the profile imparted to the notches 163 (e.g. the V-shaped opening angle imparted to them, where appropriate) may enable the determining of the final trajectory of curvature, which may be reached when the notches located on the intrados of the arc of curvature are "closed", with the opposite sides of the notch nearer to each other.

In various embodiments, the secondo tubular element 167, optionally continuous, can act as an internal core that closes the notches 163 present on the first tubular element 161; the active agent can then flow within the element 16 to be released (injected) at the site to be treated, avoiding dispersion within the vessel (e.g. blood) where the catheter has been inserted.

In various embodiments, at least one radiopaque element 170, e.g. platinum, can be associated with the flexible element 16 - for example by coupling it to the first tubular element 161 - with the function of trajectory marker of curvature to allow the operator - by means of scopy - to orient the flexible element 16 towards the lesion to be treated.

In various embodiments, the at least one trajectory marker of curvature 170 can be placed alongside at least one of said notches, for example in correspondence with at least one of the bridges 169.

In various embodiments, a plurality of trajectory markers of curvature 170 may be associated with the flexible element 16, and arranged alongside said notches 163.

In various embodiments, the flexible element 16 - straight or essentially straight when it is received within the body 12 of the catheter - is therefore able, once extracted from the inner tubular sheath 122 (and therefore no longer contained radially by the inner tubular sheath 122 itself), to become curved, or rather to take a general comma-shape.

In various embodiments, this result can be achieved, for example, when either one or the other, or both, of the outer tubular element 161 and the inner tubular element 167 presents the aforesaid curved conformation as "rest" configuration, or rather, a configuration where either one or the other, or both of these elements 161 and 167 return elastically when no longer contained by the inner tubular sheath 122 of the catheter.

In various embodiments, a similar result can be achieved, when either one or the other, or both, of the outer element 161 and the inner element 167 present characteristics of shape-memory, activatable for example by administering energy (e.g. thermal or electrical) in order to carry the element 16 into the curved condition for administering the active agent. In this case, the action of containment by the inner tubular sheath 122 of the catheter in order to maintain the element 16 in a straight condition, or essentially straight (not curved) may be unnecessary, so that it may be superfluous to envisage that the element 16 is initially sheathed and then left uncovered when curving of the element 16 is required.

In various embodiments, the structure and/or constituent materials exemplified herein allow a remote torsional control of the element 16, starting from the proximal end of the catheter (kept outside the patient's body) so as to enable rotation of the flexible element 16 about the main axis of the distal portion of the catheter (e.g. the axis of the balloon 22) to ensure administration of the active agent at the treated site.

In various embodiments, this result can be achieved by rotating even just one of the elements 161 or, respectively, 167 (having the capacity to curve - e.g. elastically and/or by shape-memory - in a determined direction) while the other element 167 or, respectively, 161 follows the first, thanks to a general flexibility.

In various embodiments, the roles of the elements 161 and 167 could be reversed with respect to what has been previously described, also without prejudice to the other characteristics previously illustrated, for example by providing the notches 163 (and therefore the flexible spinal column structure) on the inner element 167, with the outer element 161 forming a sheath or flexible coat capable of wrapping the inner element, ensuring a seal at the notches 163, which prevents leakage of the active agent through these notches.

In various embodiments, the method of use of the catheter exemplified here envisages that the catheter 10 is introduced into the body of the patient according to current modes, for example, through the femoral artery, and guided until the distal portion is located at the site to be treated (by way of example, an area of the neo-infarcted heart).

The introduction of the catheter is carried out with the balloon in the radially-contracted condition (i.e. with the balloon 22 "deflated", e.g. wrapped on the distal portion of the catheter).

Once the balloon has been carried to the treatment site, the inflation fluid is introduced under pressure into the lumen 18b, causing the balloon 22 to inflate, expanding radially, for example as shown in Figure 5, stabilizing the distal end of the catheter 10.

The flexible element 16 can be then made to exit from the body 12 of the catheter, so that the element 16 assumes, according to the criteria described above, the curved conformation exemplified in Part B of Figure 5.

In such conditions, the element 16 can be oriented in such a way to allow it to penetrate into the site (e.g. tissue lesion) to be treated. The active agent is then supplied to the flexible element 16 through the lumen 18a, in order to carry out its function.

This can occur in a concomitant, or essentially concomitant, way with an angioplasty action resulting from the dilation of the balloon 22, which may possibly be associated with an angioplasty stent in order to stabilize the stenosis.

The catheter 10 described herein is usable for percutaneous procedures intended to reach not only the coronary arteries, but also all the reachable areas through the vascular system where it may be necessary to release an active agent, e.g. a drug to treat a lesion adjacent to a vessel.

The active agent (e.g. the drug) can be used either as an aqueous solution in order to enable a rapid absorption by the lesion, or as a colloidal dispersion (for example, a gel with a viscosity of up to 200-300 cP) for obtaining a slow and controlled release at the site to be treated.

The stabilizing procedure of the distal portion of the catheter by the balloon 22 can take into account possible critical aspects of blood flow in the area. For example for stabilizing the catheter in the coronary arteries, it is possible to limit the inflation time of the balloon so as not to induce undesired side effects of cardiac ischemia.

In other areas, where the blood flow is not critical, and the consequences of a temporary blockage of the blood circulation are better tolerated, it is possible to use greater inflation times.

In various embodiments, the stabilizing balloon 22 can be mounted on the distal part of the catheter so that its axis is not aligned with that of the catheter body, so as to favor the convergence of the flexible element to the vessel wall and, consequently, to the site to be treated.

In various embodiments, the system of reaching the position of release of the active agent can be configured as in the case for systems used for angioplasty, in which the tip of the catheter is directed with a guide catheter so that it can cross particular passages, such as the coronary ostia, with the catheter made to follow beyond the ostia up to the coronary artery, making it follow a guide wire that reaches the required point.

Of course, without prejudice to the principle of the invention, the details of construction and the embodiments may be varied with respect to what is described and illustrated, without departing from the field of protection of the invention, this field of protection being defined by the attached claims.

## Claims

1. A percutaneously-insertable catheter (10) for administering an active agent to a body site, the catheter comprising:
- a distal portion (22) expandable from a contracted condition of introduction to a deployed administering condition, to stabilize the distal end (2) of the catheter (10) at said body site, and
- a flexible tubular element (16) coupled to said distal end (2) of the catheter (10) for receiving the active agent and administering it to said body site, **characterized in that**:
- the flexible element (16) comprises an outer tubular element (161) and an inner tubular element (167) inserted into the outer tubular element (161), with one (161) of said outer tubular element (161) and said inner tubular element (167) having notches (163) which enable spinal column-like curvature of the flexible element (16) towards
said body site, and
- the other (167) of said outer tubular element (161) and said inner tubular element (167) form a flexible sealing closure coat of the notches (163) present on one (161) of said outer tubular element (161) and said inner tubular element (167).

2. A catheter according to claim 1, wherein the flexible element (16) presents a sharp distal end, preferably a chamfer cut (165) of the outer tubular element (161), capable of needle-like penetration into said body site.

3. A catheter according to any one of the preceding claims, wherein said notches (163) have a V-shaped profile.

4. A catheter according to any one of the preceding claims, wherein said notches (163) are aligned on one side of said one (161) of said outer tubular element (161) and said inner tubular element (167) so as to be situated on the intrados of the arc formed by said spinal column-like curvature.

5. A catheter according to any one of the preceding claims, wherein the flexible element (16) comprises at least one marker element (170) to visualize said curvature by scopy.

6. A catheter according to any one of the preceding claims, wherein the flexible element (16) comprises a plurality of marker elements (170) to visualize said curvature by scopy, wherein said marker elements (170) are localized at said notches (163).

7. A catheter according to any one of the preceding claims, wherein said one (161) of said outer tubular element (161) and said inner tubular element (167) comprises a superelastic material, preferably nitinol.

8. A catheter according to any one of the preceding claims, wherein said other (167) of said outer tubular element (161) and said inner tubular element (167) comprises a continuous tubular body of deformable material, preferably of plastic material.

9. A catheter according to any one of the preceding claims, wherein said one (161) and said other (167) of said outer tubular element (161) and said inner tubular element (167) are, respectively, said outer tubular element (161) and said inner tubular element (167).

10. A catheter according to any one of the preceding claims, wherein the flexible element (16) is coupled to said distal end (2) of the catheter (10), with the ability to move between a retracted position within the distal end (2) of the catheter (10) and an extracted position outside of the distal end (2) of the catheter (10).

11. A catheter according to any one of the preceding claims, wherein at least one of the outer tubular element (161) and the inner tubular element (167) of the flexible element (16) is an elastic body having a curved rest configuration so that, in said extracted position outside of the distal end (2) of the catheter (10), the flexible element (16) elastically assumes a curved conformation.

12. A catheter according to any one of the preceding claims, wherein at least one of the outer tubular element (161) and the inner tubular element (167) of the flexible element (16) is a body with shape-memory characteristics, which make it assume a curved conformation.

## Patentansprüche

1. Ein perkutan einführbarer Katheter (10) zur Zuführung eines aktiven Mittels an eine Körperstelle, wobei der Katheter umfasst:
- einen distalen Bereich (22), der aus einem zusammengezogenen Einführzustand in einen eingesetzten Zuführzustand aufweitbar ist, um das distale Ende (2) des Katheters (10) an der Körperstelle zu stabilisieren, und
- ein flexibles rohrförmiges Element (16), welches mit dem distalen Ende (2) des Katheters (10) verbunden ist, um das aktive Mittel zu erhalten und es der Körperstelle zuzuführen,
**dadurch gekennzeichnet, dass**
- das flexible Element (16) ein äußeres rohrförmiges Element (161) und ein inneres rohrförmiges Element (167), welches in das äußere rohrförmige Element (161) eingesetzt ist, umfasst, wobei eines von dem äußeren rohrförmigen Element (161) und dem inneren rohrförmigen Element (167) Kerben (163) besitzt, die eine spinale säulenartige Krümmung des flexiblen Elements (16) in Richtung der Körperstelle ermöglichen, und
- das andere (167) von dem äußeren rohrförmigen Element (161) und dem inneren rohrförmigen Element (167) eine flexible abdichtende Verschlussbeschichtung der Kerben (163), die an einem (161) von dem äußeren rohrförmigen Element (161) und dem inneren rohrförmigen Element (167) vorhanden sind, bildet.

2. Ein Katheter nach Anspruch 1, **dadurch gekennzeichnet, dass** das flexible Element (16) ein scharfes distales Ende, insbesondere einen abgeschrägten Schnitt (165) des äußeren rohrförmigen Elements (161), aufweist, das für eine nadelähnliche Penetration in die Körperstelle geeignet ist.

3. Ein Katheter nach einem der vorherigen Ansprüche, **dadurch gekennzeichnet, dass** die Kerben (163) ein V-förmiges Profil haben.

4. Ein Katheter nach einem der vorherigen Ansprüche, **dadurch gekennzeichnet, dass** die Kerben (163) an einer Seite des einen (161) von dem äußeren rohrförmigen Element (161) und dem inneren rohrförmigen Element (167) in einer Linie ausgerichtet sind, so dass sie an der Innenfläche des Bogens, welcher durch die spinale säulenartige Krümmung gebildet wird, liegen.

5. Ein Katheter nach einem der vorherigen Ansprüche, **dadurch gekennzeichnet, dass** das flexible Element (16) wenigstens ein Markierungselement (170) aufweist, um die Krümmung durch Skopie zu visualisieren.

6. Ein Katheter nach einem der vorherigen Ansprüche, **dadurch gekennzeichnet, dass** das flexible Element (16) eine Mehrzahl von Markierungselementen (170) aufweist, um die Krümmung durch Skopie zu visualisieren, wobei die Markierungselemente (170) an den Kerben (163) positioniert sind.

7. Ein Katheter nach einem der vorherigen Ansprüche, **dadurch gekennzeichnet, dass** eines (161) von dem äußeren rohrförmigen Element (161) und dem inneren rohrförmigen Element (167) ein superelastisches Material, bevorzugt Nitinol, aufweist.

8. Ein Katheter nach einem der vorherigen Ansprüche, **dadurch gekennzeichnet, dass** das andere (167) von dem äußeren rohrförmigen Element (161) und dem inneren rohrförmigen Element (167) einen durchgehenden rohrförmigen Körper aus deformierbarem Material, bevorzugt aus einem Kunststoffmaterial, aufweist.

9. Ein Katheter nach einem der vorherigen Ansprüche, **dadurch gekennzeichnet, dass** das eine (161) und das andere (167) von dem äußeren rohrförmigen Element (161) und dem inneren rohrförmigen Element (167) jeweils das äußere rohrförmige Element (161) und das innere rohrförmige Element (167) sind.

10. Ein Katheter nach einem der vorherigen Ansprüche, **dadurch gekennzeichnet, dass** das flexible Element (16) mit dem distalen Ende (2) des Katheters (10) gekoppelt ist mit der Möglichkeit, sich zwischen einer zurückgezogenen Position innerhalb des distalen Endes (2) des Katheters (10) und einer ausgefahrenen Position außerhalb des distalen Endes (2) des Katheters (10) zu bewegen.

11. Ein Katheter nach einem der vorherigen Ansprüche, **dadurch gekennzeichnet, dass** wenigstens eines von dem äußeren rohrförmigen Element (161) und dem inneren rohrförmigen Element (167) des flexiblen Elements (16) ein elastischer Körper ist, der eine gekrümmte Ruhekonfiguration hat, so dass in der ausgefahrenen Position außerhalb des distalen Endes (2) des Katheters (10) das flexible Element (16) elastisch eine gekrümmte Gestalt annimmt.

12. Ein Katheter nach einer vorherigen Ansprüche, **dadurch gekennzeichnet, dass** wenigstens eines von dem äußeren rohrförmigen Element (161) und dem inneren rohrförmigen Element (167) des flexiblen Elements (16) ein Körper mit Formgedächtniseigenschaften ist, welches es dazu bringt, eine gekrümmte Gestalt anzunehmen.

## Revendications

1. Cathéter (10) pouvant être inséré par voie percutanée pour administrer un agent actif à un site corporel, le cathéter comprenant :
une partie distale (22) expansible à partir d'une condition d'introduction contractée à une condition d'administration déployée, afin de stabiliser l'extrémité distale (2) du cathéter (10) sur ledit site corporel, et
un élément tubulaire flexible (16) couplé à ladite extrémité distale (2) du cathéter (10) pour recevoir l'agent actif et l'administrer audit site corporel,
**caractérisé en ce que** :
l'élément flexible (16) comprend un élément tubulaire externe (161) et un élément tubulaire interne (167) inséré dans l'élément tubulaire externe (161), avec l'un (161) parmi ledit élément tubulaire externe (161) et ledit élément tubulaire interne (167) qui a des encoches (163) qui permettent la courbure en forme de colonne vertébrale de l'élément flexible (16) vers ledit site corporel, et
l'autre (167) parmi ledit élément tubulaire externe (161) et ledit élément tubulaire interne (167) forme un revêtement de fermeture étanche flexible des encoches (163) présentes sur l'un (161) parmi ledit élément tubulaire externe (161) et ledit élément tubulaire interne (167).

2. Cathéter selon la revendication 1, dans lequel l'élément flexible (16) présente une extrémité distale pointue, de préférence une coupe de chanfrein (165) de l'élément tubulaire externe (161), capable de pénétrer à la manière d'une aiguille dans ledit site corporel.

3. Cathéter selon l'une quelconque des revendications précédentes, dans lequel lesdites encoches (163) ont un profil en forme de V.

4. Cathéter selon l'une quelconque des revendications précédentes, dans lequel lesdites encoches (163) sont alignées d'un côté dudit un (161) parmi ledit élément tubulaire externe (161) et ledit élément tubulaire interne (167) afin d'être situées sur l'intrados de l'arc formé par ladite courbure en forme de colonne vertébrale.

5. Cathéter selon l'une quelconque des revendications précédentes, dans lequel l'élément flexible (16) comprend au moins un élément de marqueur (170) pour visualiser ladite courbure par radioscopie.

6. Cathéter selon l'une quelconque des revendications précédentes, dans lequel l'élément flexible (16) comprend une pluralité d'éléments de marqueur (170) pour visualiser ladite courbure par radioscopie, dans lequel lesdits éléments de marqueur (170) sont localisés au niveau desdites encoches (163).

7. Cathéter selon l'une quelconque des revendications précédentes, dans lequel ledit un (161) parmi ledit élément tubulaire externe (161) et ledit élément tubulaire interne (167) comprend un matériau surper élastique, de préférence du Nitinol.

8. Cathéter selon l'une quelconque des revendications précédentes, dans lequel ledit autre (167) parmi ledit élément tubulaire externe (161) et ledit élément tubulaire interne (167) comprend un corps tubulaire continu en matériau déformable, de préférence en matière plastique.

9. Cathéter selon l'une quelconque des revendications précédentes, dans lequel ledit un (161) et ledit autre (167) parmi ledit élément tubulaire externe (161) et ledit élément tubulaire interne (167) sont, respectivement, ledit élément tubulaire externe (161) et ledit élément tubulaire interne (167).

10. Cathéter selon l'une quelconque des revendications précédentes, dans lequel l'élément flexible (16) est couplé à ladite extrémité distale (2) du cathéter (10), avec la possibilité de se déplacer entre une position rétractée à l'intérieur de l'extrémité distale (2) du cathéter (10) et une position extraite à l'extérieur de l'extrémité distale (2) du cathéter (10).

11. Cathéter selon l'une quelconque des revendications précédentes, dans lequel au moins l'un parmi l'élément tubulaire externe (161) et l'élément tubulaire interne (167) de l'élément flexible (16) est un corps élastique ayant une configuration incurvée au repos de sorte que, dans ladite position extraite à l'extérieur de l'extrémité distale (2) du cathéter (10), l'élément flexible (16) prend élastiquement une conformation incurvée.

12. Cathéter selon l'une quelconque des revendications précédentes, dans lequel au moins l'un parmi l'élément tubulaire externe (161) et l'élément tubulaire interne (167) de l'élément flexible (16) est un corps avec des caractéristiques de mémoire de forme, qui lui permettent de prendre une conformation incurvée.
